(19) **Europäisches Patentamt / European Patent Office / Office européen des brevets**

(11) **EP 1 244 690 B1**

(12) **FASCICULE DE BREVET EUROPEEN**

(45) Date de publication et mention
de la délivrance du brevet:
**13.06.2007 Bulletin 2007/24**

(51) Int Cl.:
*C07K 1/34* (2006.01)   *C07K 14/26* (2006.01)
*C07K 14/205* (2006.01)

(21) Numéro de dépôt: **01903868.6**

(22) Date de dépôt: **04.01.2001**

(86) Numéro de dépôt international:
**PCT/FR2001/000023**

(87) Numéro de publication internationale:
**WO 2001/049705 (12.07.2001 Gazette 2001/28)**

(54) **PROCEDE DE PREPARATION D'UN POLYPEPTIDE SOLUBLE EN SOLVANT AQUEUX EN ABSENCE DE DETERGENT**

VERFHAREN ZUR HERSTELLUNG EINES IM WÄSSRIGEN LÖSUNGSMITTEL IN ABWESENHEIT VON DETERGENTIEN LÖSLICHEN POLYPEPTIDS

METHOD FOR PREPARING A POLYPEPTIDE SOLUBLE IN AN AQUEOUS SOLVENT IN THE ABSENCE OF DETERGENT

(84) Etats contractants désignés:
**AT BE CH CY DE DK ES FI FR GB GR IE IT LI LU MC NL PT SE TR**

(30) Priorité: **04.01.2000 FR 0000070**

(43) Date de publication de la demande:
**02.10.2002 Bulletin 2002/40**

(73) Titulaire: **PIERRE FABRE MEDICAMENT**
**92100 Boulogne-Billancourt (FR)**

(72) Inventeurs:
• **BAUSSANT, Thierry**
**F-01200 Bellegarde (FR)**
• **JEANNIN, Pascale**
**F-74160 Saint-Julien-en-Genevois (FR)**
• **DELNESTE, Yves**
**F-74160 Saint-Julien-en-Genevois (FR)**
• **LAWNY, François**
**F-74800 Saint-Sixt (FR)**
• **BONNEFOY, Jean-Yves**
**F-74350 Le Sappey (FR)**

(74) Mandataire: **Warcoin, Jacques**
**Cabinet Régimbeau**
**20, rue de Chazelles**
**75847 Paris cedex 17 (FR)**

(56) Documents cités:
**EP-A- 0 334 278**      **WO-A-96/14415**
**WO-A-97/28264**      **FR-A- 2 789 588**
**FR-A- 2 789 902**      **US-A- 5 814 455**
**US-A- 5 846 751**

Printed by Jouve, 75001 PARIS (FR)

## Description

La présente invention concerne un nouveau , procédé de préparation d'un polypeptide de la membrane externe de bactérie soluble en solvant aqueux en absence de détergent ainsi que les polypeptides présentant une structure tertiaire de type hélice α obtenus par le procédé selon l'invention. L'invention concerne également l'utilisation de tels polypeptides, en particulier pour la préparation de médicaments ou de vaccins, destinés à combattre en particulier les infections bactériennes, virales ou les cancers.

Les procédés de préparation de protéines, en particulier lorsqu'elles présentent un caractère hydrophobe ou lorsqu'elles sont produites notamment par recombinaison génétique dans les corps d'inclusion de cellules transformées, nécessitent habituellement l'addition de détergent lors des étapes d'extraction, de purification ou de renaturation, pour obtenir une protéine soluble en solvant aqueux.

On peut citer par exemple les brevets américains US 5,846,751 et US 5,814,455, qui décrivent un procédé de préparation d'antigènes pour le diagnostic issus *d'Helicobacter pylori* par un procédé comprenant une étape de solubilisation des cellules d'H. pylori en présence de détergent suivie d'une étape de chromatographie d'exclusion par taille (tamisage moléculaire), les fractions de masse moléculaire inférieure à 300 000 Da présentant une activité uréase réduite étant choisies après analyse de l'ensemble des fractions obtenues par électrophorèse PAGE-SDS en condition réduite, ainsi que la demande de brevet européen EP 0 334 278, qui décrit un procédé de purification de protéine de *Mycoplasma pneumoniae* ne comprenant pas d'étape de solubilisation en présence d'agent dénaturant.

On peut encore citer par exemple le procédé de préparation de la protéine recombinante OmpA de *Klebsiella pneumoniae* nommée rP40 décrit dans les demandes internationales de brevet WO 95/27787 ou WO 96/14415 et dans les demandes de brevet français FR 98 14007 et FR 99 01917. Pour ces procédés, la solution finale de protéine recombinante renaturée et purifiée obtenue contenait 0,1 % de zwittergent pour assurer la solubilité de la protéine.

Cependant, lorsque de telles protéines sont destinées en particulier à être injectées in vivo, la présence de ces détergents est potentiellement toxique (risques de nécrose, ...).

D'autre part, de tels procédés de préparation de protéines, notamment recombinantes, nécessitant l'addition de détergent afin d'augmenter leur solubilité en solvant aqueux confèrent à ces protéines une structure tertiaire qui selon la nature de la protéine préparée n'est pas la structure tertiaire permettant d'obtenir la meilleure activité pour cette protéine, comme mis en évidence ci-après dans les exemples.

Ainsi, il existe aujourd'hui un besoin de disposer d'un procédé de préparation de protéine permettant d'obtenir une protéine soluble en solvant aqueux, notamment après renaturation, en l'absence ou en quantité non significative de détergent, et le cas échéant présentant une structure tertiaire différente de celle obtenue par un procédé de préparation de protéine nécessitant la présence finale de détergent pour augmenter sa solubilité en solvant aqueux, ladite structure tertiaire différente permettant d'obtenir une meilleure activité.

Ceci est justement l'objet de la présente invention.

La présente invention concerne un procédé de préparation d'une solution purifiée de polypeptide soluble en solvant aqueux en absence de détergent à partir d'une solution purifiée d'un polypeptide hydrophobe contenant un détergent, ledit polypeptide étant une protéine de la membrane externe de bactérie, caractérisé en ce qu'il comprend les étapes suivantes :

a) élimination dudit détergent de ladite solution purifiée contenant un détergent
b) solubilisation du polypeptide obtenu à l'étape a) dans une solution contenant un agent dénaturant choisi parmi l'urée ou le chlorhydrate de guanidine;
c) élution en milieu aqueux du polypeptide solubilisé à l'étape b) par tamisage moléculaire sur colonne de chromatographie.

Par "polypeptide", on entend également désigner un peptide ou une protéine, ces trois termes étant utilisés indifféremment dans la présente description.

De manière surprenante, les inventeurs ont montré que le nouveau procédé de préparation selon l'invention utilisant une étape de chromatographie de tamisage moléculaire dans l'eau, permet d'obtenir une protéine renaturée, notamment une protéine membranaire recombinante, soluble dans l'eau en l'absence de tout détergent, notamment potentiellement toxique lors d'injection *in vivo.*

Les polypeptides purifiés en présence de détergent selon l'invention sont les protéines Omp localisées dans la membrane externe de bactéries (Omp pour "Outer Membrane Protein"), telles que la P40 naturelle (Omp de typeA) de *Klebsiella pneumoniae.*

Lorsque ledit polypeptide est un polypeptide produit par synthèse chimique, on préfère les polypeptides présentant une forte homologie avec les protéines de la membrane externe de bactéries Gram négatives, notamment les polypeptides dont la séquence forme des feuillets β de nature hydrophobe.

Dans le procédé selon l'invention, ledit polypeptide purifié contenu dans ladite solution purifiée d'un polypeptide contenant un détergent est un polypeptide hydrophobe (ou à caractère hydrophobe).

Par "polypeptide hydrophobe (ou à caractère hydrophobe)", on entend désigner un polypeptide dont la séquence comprend un ou plusieurs domaines hydrophobes. De tels domaines hydrophobes peuvent être par exemple déterminés en utilisant l'algorithme de Kyte et Doolittle (J. Mol. Biol., 157, 105-132, 1982).

De préférence, par protéine hydrophobe, on entend désigner ici un polypeptide dont la forme native, recombinante ou obtenue après synthèse chimique, ou encore renaturée, n'est pas soluble en solvant aqueux (tel que l'eau ou en solution saline) ou n'est pas soluble en solvant aqueux en quantité suffisante pour être utilisée notamment dans des compositions pharmaceutiques en l'absence de détergent.

De manière plus préférée, par protéine hydrophobe, on entend désigner ici un polypeptide soluble en solvant aqueux en présence de détergent ou d'agent dénaturant tel que l'urée ou le chlorhydrate de guanidine, ladite protéine hydrophobe n'étant pas soluble ou insuffisamment soluble en solvant aqueux en absence de détergent.

Par "renaturé", on entend désigner un polypeptide possédant des propriétés biologiques similaires au polypeptide natif, c'est-à-dire qu'elle peut être diminuée, égale ou amplifiée, mais que, hors ces considérations d'échelle, les conséquences de l'ajout d'un polypeptide ou d'un autre dans un échantillon biologique sont semblables. Ainsi, on concevra que les structures tertiaires du polypeptide renaturé selon la définition et du polypeptide natif puissent être différentes.

Dans un mode de réalisation préféré, l'invention comprend un procédé selon l'invention, caractérisé en ce que ledit polypeptide purifié contenu dans ladite solution purifiée d'un polypeptide contenant un détergent est un polypeptide recombinant.

Ledit polypeptide recombinant est choisi parmi les polypeptides recombinants dont la forme native est de nature hydrophobe, non solubles en solvant aqueux ou dont on cherche à augmenter la solubilité en solvant aqueux et/ou parmi les polypeptides recombinants hydrophobes produits dans des corps d'inclusion cytoplasmique de la cellule hôte transformée.

Dans un mode de réalisation préféré, l'invention comprend un procédé selon l'invention, caractérisé en ce que ledit polypeptide purifié contenu dans ladite solution purifiée d'un polypeptide contenant un détergent est un polypeptide dont la stucture tertiaire est de type en feuillet β, structure tertiaire correspondant notamment à la forme de la protéine native et/ou à la forme de la protéine recombinante, le cas échéant après renaturation.

Dans un mode de réalisation également préféré, ledit polypeptide recombinant est choisi parmi les polypeptides recombinants dont la stucture tertiaire de la forme native est de type en feuillet β.

L'étape a) d'élimination du détergent du procédé peut être effectuée par précipitation, notamment par addition d'alcool organique, mais également par dialyse ou par gel filtration en alcool organique.

L'étape b) de solubilisation du polypeptide obtenu à l'étape a) du procédé peut être effectuée par reprise du précipité, notamment par un agent dénaturant, mais également par dialyse ou par gel filtration dans ces agents dénaturants, tels que l'urée ou le chlorhydrate de guanidine.

L'invention comprend de préférence un procédé selon l'invention, caractérisé en ce que l'étape a) d'élimination du détergent de ladite solution purifiée est effectuée par précipitation dudit polypeptide en présence d'un alcool organique, de préférence l'éthanol.

Dans un mode de réalisation également préféré, l'invention comprend un procédé selon l'invention, caractérisé en ce que le polypeptide est une protéine de la membrane externe de bactéries Gram-négatives.

Dans un mode de réalisation encore plus préféré, l'invention comprend un procédé selon l'invention, caractérisé en ce que ledit polypeptide est la protéine OmpA de *Klebsiella pneumoniae* de séquence SEQ ID No. 2 ou une protéine hydrophobe dont la séquence présente un pourcentage d'identité d'au moins 80 %, 85 %, 90 %, 95 % ou 99 % après alignement optimal avec la séquence SEQ ID No. 2.

Par "pourcentage d'identité" entre deux séquences d'acides aminés au sens de la présente invention, on entend désigner un pourcentage de résidus d'acides aminés identiques entre les deux séquences à comparer, obtenu après le meilleur alignement, ce pourcentage étant purement statistique et les différences entre les deux séquences étant réparties au hasard et sur toute leur longueur. Le meilleur alignement ou alignement optimal est celui pour lequel le pourcentage d'identité entre les deux séquences à comparer déterminé comme ci-après est le plus élevé. Les comparaisons de séquences entre deux séquences d'acides aminés sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou BLASTP).

Le pourcentage d'identité entre deux séquences d'acides aminés est déterminé en comparant ces deux séquences alignées de manière optimale, la séquence d'acides aminés à comparer pouvant comprendre des additions ou des délétions par rapport à la séquence de référence pour obtenir l'alignement optimal entre ces deux séquences. Le pourcentage d'identité est calculé en déterminant le nombre de positions identiques pour lesquelles le résidu d'acide aminé est identique entre les deux séquences, en divisant ce nombre de positions identiques par le nombre total de positions comparées et en multipliant le résultat obtenu par 100 pour obtenir le pourcentage d'identité entre ces deux séquences.

Le logiciel BLAST est habituellement utilisé par les inventeurs et par l'homme de l'art pour comparer et déterminer le pourcentage d'identité entre deux séquences d'acides aminés. Les paramètres tels que le "gap cost" et la matrice de substitution sont directement sélectionnés par le logiciel en fonction de la longueur des séquences à comparer. Par exemple, on pourra utiliser le programme BLAST, "BLAST 2 séquences", disponible sur le site http://www.ncbi.nlm.nih.gov/gorf/bl2.html, les paramètres utilisés étant ceux donnés par défaut (en particulier pour les paramètres "open gap penaltie" : 5, et "extension gap penaltie": 2 ; la matrice choisie étant par exemple la matrice "BLOSUM 62" proposée par le programme), le pourcentage d'identité entre les deux séquences à comparer étant calculé directement par le programme.

L'invention comprend également un procédé selon l'invention, pour la modification de la structure tertiaire de type feuillet β d'une protéine recombinante hydrophobe en structure tertiaire de type hélice α.

De manière également surprenante, les inventeurs ont mis en évidence que la protéine recombinante obtenue selon le procédé de préparation selon l'invention conserve les propriétés biologiques de la protéine rP40 purifiée comme décrit dans les demandes internationales de brevet WO 95/27787 ou WO 96/14415, et présente même certaines activités biologiques qui sont amplifiées.

La protéine recombinante P40 obtenue selon le procédé de préparation selon l'invention (dénommée rP40s, "s" pour soluble) est sous forme stable, avec des hélices α, et est hydrophile, un résultat inattendu, dans la mesure ou la protéine native est hydrophobe, et présente une structure en feuillets β. Toutefois, et comme les exemples le démontreront, les deux protéines possèdent une activité biologique similaire, l'activité de la protéine obtenue suivant le procédé selon l'invention étant même supérieure à celle observée pour la protéine native, ceci pouvant être dû au fait que le procédé selon l'invention permet l'obtention d'une protéine qui présente une nouvelle structure tertiaire qui n'est pas observée de façon générale, et en particulier qui n'existe pas dans la nature. Cette protéine possédant de telles propriétés, est donc également l'un des objets de l'invention.

Ainsi, l'invention comprend les polypeptides de la membrane externe de bactérie, recombinants, renaturés et solubles dans l'eau, obtenus par le procédé selon l'invention, en particulier la protéine P40 de *Klebsiella pneumoniae* de séquence SEQ ID No. 2 ou d'une protéine dont la séquence présente un pourcentage d'identité d'au moins 80 %, 85 %, 90 %, 95 % ou 99 % avec la séquence SEQ ID No. 2, caracterisés en ce que lesdits polypeptides présentent une structure tertiaire de type hélice α.

La protéine rP40 est une OmpA issue de la membrane externe de *Klebsiella pneumoniae.* L'extraction et la purification de cette protéine ont été précédemment décrites dans les demandes de brevet WO 95/27787 et WO 96/14415.

Cette protéine possède des propriétés très intéressantes d'un point de vue immunologique, en particulier pour les activités suivantes :

- protéine porteuse capable d'induire une réponse anticorps (AC) dirigée contre un haptène (polysaccharidique ou peptidique) (cf. demandes de brevet WO 95/27787 et WO 96/14415),
- favorise le développement d'une réponse cytotoxique vis-à-vis des antigènes avec lesquels elle est co-injectée (cf. demande de brevet FR 99 01917),
- se fixe sélectivement sur les cellules présentatrices d'antigènes (CPAg) telles que les cellules dendritiques (CD) (cf. demande de brevet FR 98 14007).

Ainsi, cette protéine présente un intérêt inestimable en thérapeutique afin de générer une réponse lymphocytaire B (et en particulier des AC spécifiques) ou une réponse lymphocytaire T cytotoxique vis-à-vis d'une molécule donnée, qui est introduite dans l'organisme en même temps que rP40 (associée ou en mélange). De plus, de par sa capacité à se fixer sélectivement aux CPAg et en particulier aux cellules dendritiques, rP40 est utilisable pour favoriser le ciblage, la présentation et/ou l'expression de molécules par les CPAg. Ces cellules étant à l'origine de l'initiation des réponses lymphocytaires B et T spécifiques, rP40 est donc un outil considérable permettant de générer une réponse spécifique de l'organisme vis-à-vis d'une molécule donnée.

La protéine rP40 possède donc un intérêt dans toutes les pathologies où il s'avère bénéfique d'induire ou d'amplifier une réponse immune spécifique inexistante ou inefficace, en particulier pour une des utilisations suivantes :

(i) en vaccinologie thérapeutique, en particulier anticancéreuse ou prophylactique, en particulier anti-infectieuse (Virus, bactéries, parasites, fongiques), et
(ii) lors d'infections chroniques ou récurrentes. Sa capacité à se fixer aux CD élargit son domaine d'utilisation aux diverses pathologies dans lesquelles le ciblage des CPAg avec une molécule chimique ou biologique présente un intérêt. On peut citer en particulier les maladies auto-immunes, rejets de greffes, maladies cardiovasculaires, maladies inflammatoires, maladies infectieuses ou liées à une immunodéficience.

Les CD jouent un rôle crucial dans le développement d'une réponse immune et dans l'initiation d'une réponse lymphocytaire T spécifique (Steinman RM. et al., Immuno. Rev. (1997) 156, 25, et Sella M. et al., Curr. Opin. Immunol. (1997) 9, 10). Au niveau périphérique, les cellules dendritiques sont immatures et captent et digèrent les antigènes de façon très efficace. Après une stimulation antigénique *in vivo* ou une stimulation par des molécules pro-inflammatoires, les CD qui ont capté les antigènes migrent dans les organes lymphoïdes. Durant cette migration, les CD subissent des modifications fonctionnelles et phénotypiques qui sont regroupées sous le terme de maturation. Cette maturation se caractérise par une augmentation à leur surface de molécules impliquées dans l'activation des lymphocytes T (telles que CD40, CD54, CD58, CD86 et CMH de classe I/II), la production de cytokines (telles que TNF$\alpha$ et IL-12) et l'induction de l'expression en surface de molécules spécifiques (comme le CD83 pour les cellules humaines) et la perte de leur capacité à processer l'antigène. Dans les zones thymodépendantes des organes lymphoïdes, les CD qui ont migré ont acquis de puissantes propriétés immunostimulatrices et vont donc activer de façon très efficace les lymphocytes T naïfs circulants.

Ainsi, des molécules permettant non seulement de cibler les CD mais également d'induire la maturation de ces cellules présentent un intérêt considérable en thérapeutique afin d'induire une réponse immune spécifique vis-à-vis d'un antigène donné.

Sous un autre aspect, l'invention a ainsi pour objet l'utilisation d'un polypeptide selon la présente invention, seul ou en tant qu'adjuvant, pour la préparation d'une composition thérapeutique soluble dans l'eau en absence de détergent.

L'invention concerne également l'utilisation dudit polypeptide selon l'invention, en combinaison avec un antigène ou un haptène, pour la préparation d'un médicament destiné à moduler la réponse immunitaire de l'hôte vis-à-vis dudit antigène ou haptène.

L'antigène ou l'haptène pourra être tout composé d'intérêt thérapeutique.

L'haptène est choisi dans le groupe comprenant les peptides, les lipopeptides, les polysaccharides, les oligosaccharides, les acides nucléiques, les lipides ou toute molécule chimique capable d'induire une réponse immune dirigée spécifiquement contre ledit antigène ou haptène.

L'antigène ou haptène pourra être utilisé en combinaison avec le polypeptide selon l'invention, soit dans un simple mélange, soit après couplage audit polypeptide, en particulier par une liaison chimique, de préférence de type liaison covalente. La liaison covalente pourra en particulier être obtenue par production de protéines hybrides, le polypeptide ainsi obtenu étant alors renaturé par le procédé selon l'invention.

Ainsi, de manière préférée, l'utilisation dudit polypeptide selon l'invention est caractérisée en ce que l'antigène ou haptène est couplé audit polypeptide par une liaison chimique, de préférence de type liaison covalente.

Lesdits polypeptides obtenus pourront être utilisés suivant un procédé selon l'invention dans de nombreuses applications, en particulier en thérapeutique humaine ou animale, de façon préférée, comme cela a été déjà discuté, dans toute application destinée à moduler le système et/ou la réponse immunitaire chez un mammifère.

L'invention comprend en outre l'utilisation dudit polypeptide selon l'invention pour la préparation d'un vaccin, en particulier antiviral, antibactérien ou anticancéreux.

L'invention, décrit également un procédé de modulation du système immunitaire d'un mammifère vis-à-vis d'un antigène, en particulier bactérien, viral ou tumoral, caractérisé en ce qu'il comprend les étapes suivantes :

a) isolement des cellules dendritiques immatures d'un mammifère ;
b) induction de la maturation desdites cellules isolées à l'étape a) par incubation en présence d'un polypeptide selon l'invention ;
c) incubation des cellules obtenues à l'étape b) en présence dudit antigène, ou transformation desdites cellules avec un vecteur exprimant ledit antigène ;
d) injection des cellules obtenues à l'étape c) dans un mammifère, pouvant être le mammifère de l'étape a).

L'invention décrit aussi un procédé de modulation du système immunitaire d'un mammifère caractérisé en ce qu'il comprend une étape d'injection d'un polypeptide selon l'invention, seul ou en tant qu'adjuvant.

Les propriétés observées pour les polypeptides selon l'invention, en particulier pour la protéine rP40s, renaturée suivant un procédé selon l'invention pourront être optimisées par l'isolement de cellules dendritiques d'un mammifère, la maturation desdites cellules dendritiques *ex vivo* en utilisant un polypeptide selon l'invention, suivie de leur chargement par des antigènes particuliers, soit de façon externe, soit après transfection avec un plasmide codant pour un ou des antigène(s) d'intérêt, et la réinjection de ces cellules chargées en antigène(s) dans la circulation dudit mammifère ou d'un autre mammifère.

Ce procédé de modulation du système immunitaire pourra être mis en oeuvre en particulier en cas de cancer, pour des tumeurs faiblement immunogènes, ou pour toute autre maladie bactérienne, virale fongique et/ou parasitaire, chronique ou récurrente, caractérisée en particulier par une réponse immunitaire peu efficace. On pourra citer, sans que cette liste ne soit limitative, l'infection au VIH, au virus syncytial respiratoire, au virus de la rougeole, des oreillons, les hépatites, la tuberculose, les mycoses, ....

Sous un dernier aspect, la présente invention concerne une composition thérapeutique comprenant dans un milieu pharmaceutiquement acceptable au moins un polypeptide obtenu par un procédé de préparation selon l'invention, ledit polypeptide étant la protéine OmpA de SEQ ID No.2 ou de séquence identique à au moins 80%.

De manière préférée, la composition thérapeutique selon l'invention comprend dans un milieu pharmaceutiquement acceptable au moins ce polypeptide OmpA préparé selon l'invention en combinaison avec un antigène ou un haptène.

L'invention comprend en outre une composition thérapeutique selon l'invention en ce qu'elle est exempte de détergent.

Les légendes des figures ci-après ainsi que les exemples qui suivent sont destinés à illustrer l'invention sans aucunement en limiter la portée.

## Légendes des figures

**Figure 1 : Comparaison de la masse apparente de rP40 et rP40s non chauffées sur gel de polyacrylamide.**
Une électrophorèse sur gel de polyacrylamide SDS PAGE a été effectuée sur les protéines non chauffées. Colonne 1 : marqueur de masse moléculaire ; colonne 2 : rP40 ; colonne 3 : rP40s.

**Figure 2 : rP40s induit l'expression de CD83 sur les CD humaines.**
Des CD générées à partir des monocytes provenant de 2 sujets (□ et ■) ont été exposées à différentes concentrations de rP40s. Après 4 jours, l'expression de CD83 a été évaluée par FACS. Les résultats sont exprimés en pourcentage de cellules exprimant CD83.

**Figure 3 : rP40s induit une production d'interleukine (IL)-12 par les CD humaines.**
Des CD générées à partir des monocytes provenant de 3 sujets (■, □ et ▣) ont été exposées à différentes concentrations de rP40s. Après 2 jours, la production d'IL-12 a été évaluée par FACS dans les surnageants. Les résultats sont exprimés en pg/ml.

**Figure 4 : La production d'IL-12 induite par rP40s n'est pas inhibée par la polymixine B.**
Des CD générées à partir des monocytes provenant de 3 sujets (exp. 1, exp. 2 et exp. 3) ont été exposées à différentes concentrations de rP40s en l'absence (■) ou présence (□) de 5 $\mu$g/ml de polymixine B. En tant que contrôle, les CD d'un sujet ont été exposées à 1 ng/ml de lypopolyssacharide (LPS) en absence (■) ou en présence (□) de 5 $\mu$g/ml de polymixine B. Après 2 jours, la production d'IL-12 a été évaluée par FACS dans les surnageants. Les résultats sont exprimés en ng/ml.

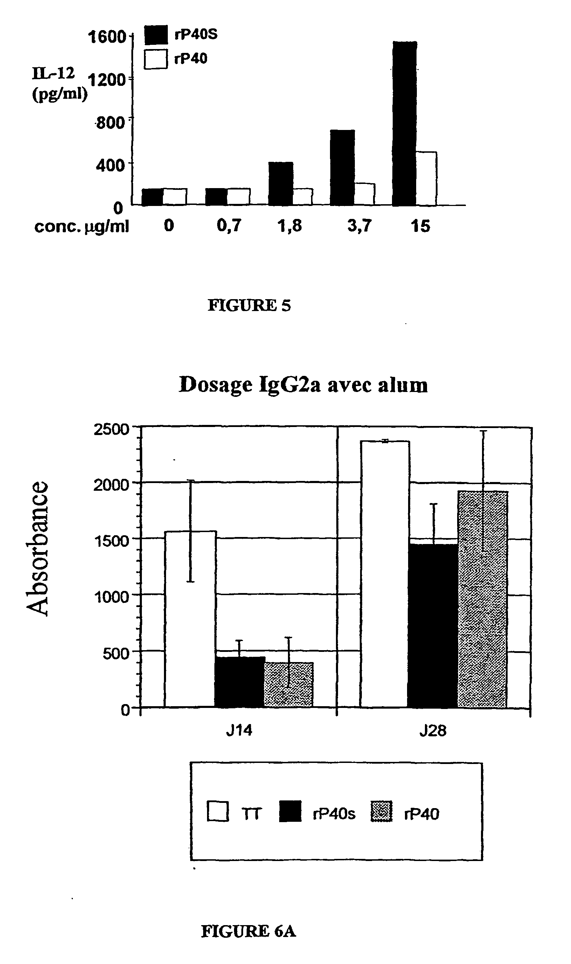

**Figure 5 : rP40s induit une production d'IL-12 par les DC plus importante que rP40.**
Les CD générées à partir des monocytes provenant d'un sujet ont été exposées à différentes concentrations de rP40s (■) et de rP40 (□). Après 2 jours, la production d'IL-12 a été évaluée par FACS dans les surnageants. Les résultats sont exprimés en pg/ml.

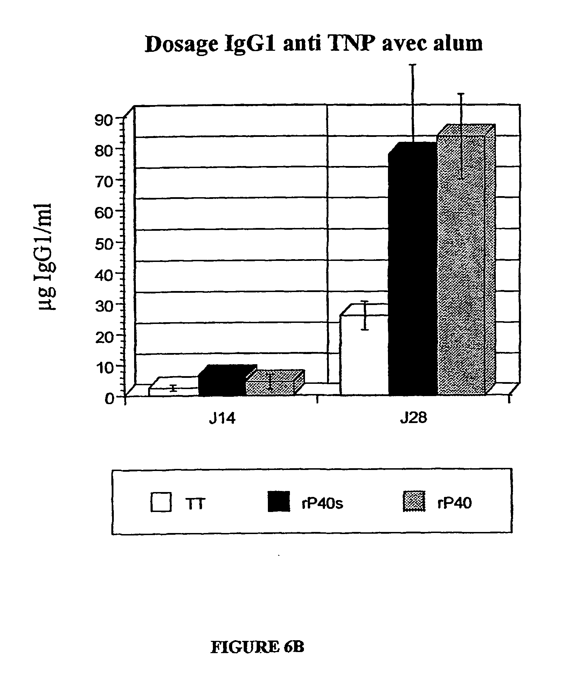

**Figures 6A et 6B : Induction d'une réponse anticorps anti-haptène.**
Comparaison des taux d'anticorps anti-TNP pour les conjugués rP40s-TNP (■) ou les conjugués rP40-TNP (▣). TT (□) : anatoxine tétanique-TNP. La figure 6A montre les réponses IgG2a exprimées en absorbance pour une dilution au 2500ème de sérum. La Figure 6B montre les réponses IgG1 exprimées en $\mu$g d'anticorps par ml de sérum.

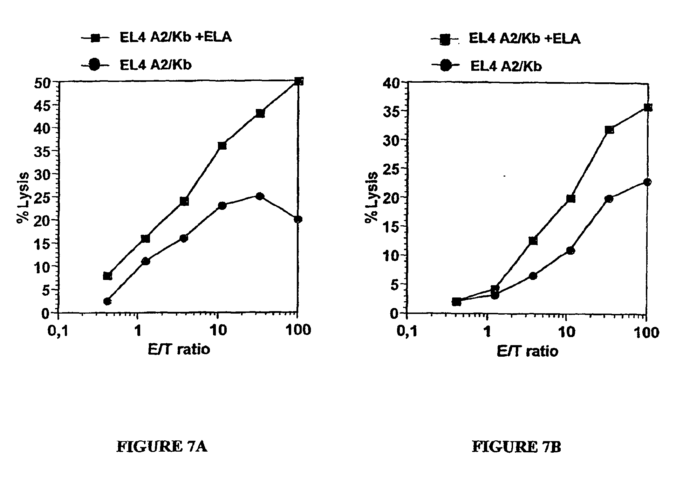

**Figures 7A et 7B : Test comparatif de P40 soluble (P40S, figure 7B) versus P40 Zwittergent (P40Z, figure 7A) pour l'induction d'une réponse CTL dirigée contre le peptide ELA**
Les figures 7A et 7B représentent le pourcentage de lyse obtenu (pourcentage de cytotoxicité), calculé par la formule: 100 X (Lyse de l'essai - Lyse spontanée / Lyse totale - Lyse spontanée) en fonction d'une gamme de cellules effectrices (exprimée en pourcentage de cellules effectrices sur cellules totales E/T).
Les figures 7A et 7B représentent les courbes ainsi obtenues pour les cellules EL4 A2/Kb chargées (■) ou non chargées (●) avec le peptide ELA, les souris HLA A2/Kb ayant été préalablement immunisées en présence de P40Z (figure 7A) ou P40S (figure 7B).

## Exemples

### Exemple 1 : Clonage du gène rP40 dans un vecteur d'expression

Le gène codant pour rP40 a été obtenu par amplification par PCR à partir de l'ADN génomique de *Klebsiella pneumoniae* IP I145 (Nguyen et col., Gene, 1998). Le fragment de gène codant de rP40 est inséré dans divers vecteurs d'expression, en particulier un vecteur sous le contrôle du promoteur de l'opéron Trp. La séquence nucléotidique et la séquence peptidique déduite de rP40 sont représentées dans la liste des séquences respectivement par la séquence SEQ ID No. 1 et SEQ ID No. 2. Une souche *productrice E coli* K12, a été transformée par un vecteur d'expression pvaLP40. La protéine rP40 est produite sous forme de corps d'inclusion avec un rendement important (> 10 %, g de protéines / g de biomasse sèche). Cet exemple n'est qu'une illustration de l'expression de la rP40, mais elle peut être étendue à d'autres souches bactériennes ainsi que d'autres vecteurs d'expression.

### Exemple 2 : Procédé de fermentation de protéines de fusion rP40

Dans un erlenmeyer contenant 250 ml de milieu TSB (Tryptic Soy Broth, Difco), de l'ampicilline (100 $\mu$g/ml, Sigma) et de la tétracycline (8 $\mu$g/ml, Sigma), on inocule avec la souche *E. coli* recombinante décrite dans l'exemple 1. On incube pendant une nuit à 37°C puis 200 ml de cette culture servent à ensemencer 2 litres de milieu de culture dans un fermenteur (Biolafitte, France). De manière assez classique, le milieu de culture peut être composé d'agents chimiques, supplémentés par des vitamines et/ou des extraits de levure. Les différents suppléments utilisés seront optimisés par l'homme du métier qui désire induire une croissance élevée des cellules bactériennes.
Les paramètres contrôlés durant la fermentation sont : le pH, l'agitation, la température, le taux d'oxygénation et l'alimentation en sources carbonées (glycérol ou glucose). De manière générale, le pH est régulé à 7,0 et la température fixée à 37°C. La croissance est contrôlée en alimentant la culture en glycérol (87 %) à un débit constant (12 ml/h) pour maintenir le signal de tension de l'oxygène dissous à 30 %. Lorsque la turbidité de la culture (mesurée à 580 nm) atteint la valeur de 80 (après environ 24 heures de culture), la production des protéines est déclenchée par addition de l'acide indole acrylique (IAA) à la concentration finale de 25 mg/l. Environ 4 heures après l'induction, les cellules sont récoltées par centrifugation. On obtient environ 200 g de quantité de biomasse humide.

### Exemple 3 : Procédé d'extraction et de purification de la protéine rP40

#### Extraction de rP40

Après centrifugation du bouillon de culture (4000 rpm, 10 min, 4°C), les cellules sont remises en suspension dans un tampon Tris-HCl 25 mM pH 8,5. Les corps d'inclusion sont obtenus après lyse des bactéries par le lysozyme (0,5 g/litre, 1 heure à température ambiante / agitation douce). Le culot de corps d'inclusion obtenu par centrifugation (15 min à 10 000 g à 4°C) est repris dans un tampon Tris-HCl 25 mM à pH 8,5 et 5 mM MgCl$_2$, puis centrifugé (15 min à 10 000 g). Les corps d'inclusion sont solubilisés par traitement avec une solution Tris-HCl 25 mM pH 8,5 contenant 7 M urée (agent dénaturant) et 10 mM de Dithiothréitol (réduction des ponts disulfures) pendant 2 heures à 37°C. Une centrifugation de 15 min à 10 000 g permet d'éliminer les particules non solubles.
La suspension est mélangée avec 13 volumes de tampon Tris-HCl 25 mM pH 8,5 contenant du NaCl (8,76 g/l) et du Zwittergent 3-14 (0,1 %, p:v). La solution est laissée pendant une nuit à température ambiante sous agitation douce au contact de l'air afin de favoriser la renaturation des protéines par dilution et réoxydation des ponts disulfures.

#### Purification de la protéine rP40

#### Etape de chromatographie d'échange d'anions

Après une nouvelle centrifugation, la solution est dialysée contre 100 volumes de tampon Tris-HCl 25 mM pH 8,5 contenant 0,1 % Zwittergent 3-14 pendant une nuit à 4°C.
Le dialysat est déposé sur une colonne de chromatographie contenant un support de type échangeur d'anions forts (gel Macro Prep High Q, Biorad) équilibrée dans le tampon décrit ci-dessus à un débit linéaire de 15 cm/h Les protéines sont détectées à 280 nm. La protéine rP40 est éluée, avec un débit linéaire de 60 cm/h, pour une concentration de 0,2 M en NaCl dans le tampon Tris-HCl 25 mM, pH 8,5 ; 0,1 % Zwittergent 3-14.

#### Etape de chromatographie d'échange de cations

Les fractions contenant la protéine rP40 sont mélangées puis concentrées par ultrafiltration à l'aide d'un système de cellule à agitation Amicon utilisé avec une membrane Diaflo de type YM10 (seuil de coupure 10 kDa) pour des volumes de l'ordre de 100 ml, ou à l'aide d'un système de filtration à flux tangentiel Minitan Millipore utilisé avec des plaques de membranes possédant un seuil de coupure 10 kDa pour des volumes supérieurs. La fraction concentrée est dialysée pendant une nuit à 4°C contre un tampon citrate 20 mM pH 3,0 contenant 0,1 % de Zwittergent 3-14.

Le dialysat est déposé sur une colonne contenant un support de type échangeur de cations forts (gel Biorad Macro Prep High S) équilibrée dans le tampon citrate 20 mM pH 3,0, à 0,1 % de Zwittergent 3-14. La protéine rP40 est éluée (vitesse 61 cm/h) pour une concentration 0,7 M en NaCl. Les profils électrophorétiques montrent un degré de pureté de l'ordre de 95%. L'état de la protéine est suivi par SDS-PAGE.

## Exemple 4 : mise en oeuvre du procédé selon l'invention. Obtention de protéine rP40s

### Elimination du zwittergent 3-14 par précipitation de rP40 à l'éthanol

De l'éthanol froid (- 20°C) est ajouté à la solution purifiée de rP40 (environ 4 mg/ml) dans un rapport de 5 pour 1. Après une heure à 4°C, la solution est centrifugée 10 min à 10 000g. Le précipitat est repris dans une solution d'urée 7M dans l'eau pour atteindre une concentration en rP40 de 2 mg/ml.

### Renaturation de rP40 dans l'eau par tamisage moléculaire

La solution de rP40 dans l'urée 7M est déposée sur une colonne de Fractogel EMD Biosec (volume déposé : 5% du volume de la colonne). La rP40s est éluée à une vitesse linéaire de 30 cm/h. Le pic contenant la rP40s est collecté et l'éluat est stérilisé par filtration et stocké à 4°C.

Selon sa structure tertiaire, la protéine rP40 possède un comportement électrophorétique (migration) caractéristique. La forme native hydrophobe (structure en feuillets β) présente en effet une masse moléculaire apparente plus faible que la forme présentant une structure en hélices α. On observe que la protéine rP40s obtenue suivant le procédé selon l'invention est sous forme hélice α, hydrophile et stable.

### Mise en évidence des modifications de conformation entre rP40 et rP40s

rP40, l'OmpA de *Klebsiella pneumoniae,* présente la propriété de changer de masse apparente en SDS-PAGE suivant l'état conformationnel de la protéine ("heat modifiable protein", voir Rosenbusch, J. Biol. Chem. (1974), 249: 8019-8029). Sans chauffage à 100°C en présence du tampon de reprise SDS-PAGE, la protéine native, constituée de feuillets β, présente une masse apparente de 32 kDa environ alors que la protéine dénaturée à la chaleur présente une masse apparente de 38 kDa. La forme dénaturée adsorbe moins de SDS, ce qui explique cette migration réduite dans le gel d'électrophorèse. Cette adsorption réduite de SDS est due à des structures en hélice α (Nakamura et Mizushima, J. Biochem, (1976), 80:1411-1422).

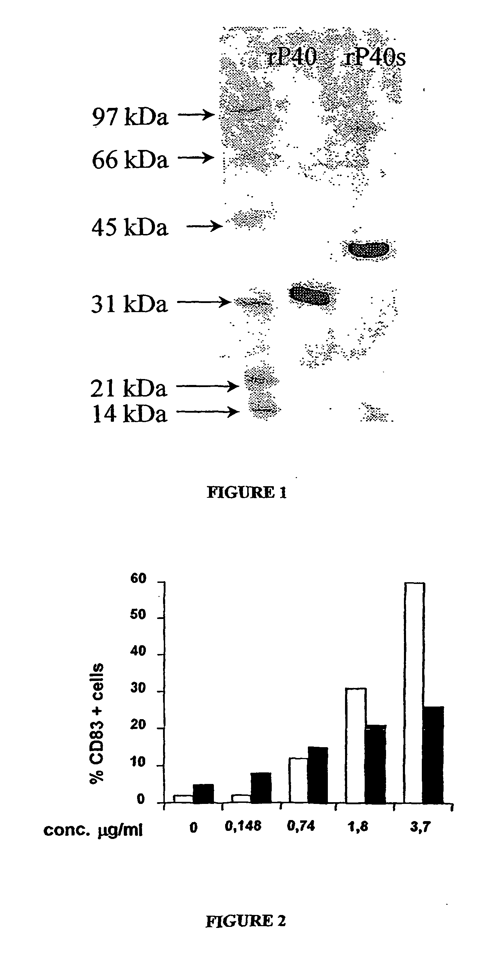

Comme le montre la figure 1, la rP40 non chauffée, comme préparée à l'exemple 3, présente une masse apparente de 32 kDa (feuillets β) alors que la rP40s, non chauffée également, présente une masse apparente de 38 kDa, caractéristique de structures en hélice α.

## Exemple 5 : Induction de la maturation des cellules dendritiques humaines par rP40s

L'exemple 5 montre que rP40s induit l'expression de la molécule CD83 et la production d'IL-12 par les CD humaines. Ces deux paramètres sont associés au processus de maturation des CD humaines. En particulier, l'expression de la molécule CD83 caractérise les CD matures, cette molécule n'étant pas exprimée sur les CD immatures.

### Induction de l'expression de la molécule CD83 sur les CD humaines

### - Isolement in vitro de cellules dendritiques humaines immatures

Les cellules dendritiques humaines sont isolées à partir de monocytes isolés du sang périphérique. Le sang est prélevé par leucophérèse en présence d'anticoagulant comme par exemple l'héparinate de lithium. Les cellules mononucléées (CMN) sont isolées de sujets sains par centrifugation sur un gradient de Ficoll-Hypaque (densité = 1,077) (Amersham Pharmacia Biotech, Uppsala, Suède). Brièvement, les cellules du sang sont centrifugées à 1 500 rpm pendant 30 minutes à température ambiante. Les CMN, localisées à l'interface Ficoll-plasma, sont récupérées et lavées deux fois en présence de milieu RPMI 1640 (Life Technologies, Cergy-Pontoise, France).

Les monocytes sont purifiés par sélection positive en utilisant un séparateur magnétique de cellules (MACS™ ; Miltenyi Biotex, Bergisch Gladbach, Allemagne) en accord avec les instructions du manufactureur. Brièvement, les CMN sont incubées pendant 20 minutes à 4°C avec des billes magnétiques sur lesquelles est fixé un anticorps monoclonal anti-CD14 humain. Après lavage, la suspension cellule plus billes est déposée sur une colonne et soumise à un champ magnétique. Après trois lavages, la colonne n'est plus soumise au champ magnétique et les monocytes sont collectés par gravitation. La pureté des monocytes est évaluée par cytofluorométrie (cytofluoromètre FACScan ; Becton Dickinson, Erembodegem, Belgique) sur la base des paramètres taille-granulosité des cellules. La pureté est supérieure à 98 %.

Les monocytes sont ensuite mis en culture à la concentration de 5 x $10^6$ cellules/ml dans le milieu suivant (dénommé par la suite milieu de culture complet) : milieu RPMI 1640 supplémenté de 10 % de sérum de veau foetal décomplémenté (chauffage à 56°C pendant 30 minutes), 2 mM de L-glutamine, 50 U/ml de pénicilline et 50 $\mu$g/ml de streptomycine (Life technologies) dans des plaques de culture 6 puits (Nunc, Roskilde, Danemark) à raison de 5 ml de milieu par puits. Les cellules sont activées avec 20 ng/ml d'IL-4 humaine recombinante et 20 ng/ml de GM-CSF humaine recombinante (R & D Systems, Abingdon, Royaume Uni).

Après 6 jours de culture (37°C, 5 % $CO_2$ en atmosphère humide), le phénotype des cellules est défini par cytofluorométrie. Brièvement, un aliquote de la suspension cellulaire est prélevé. Les cellules sont lavées dans du tampon FACS (tampon phosphate 10 mM pH 7,4 contenant 1 % de sérum albumine bovine et 0,01 % d'azide de sodium) puis réparties dans des puits d'une plaque de culture 96 puits à fond conique (Nunc) à raison de 2 x $10^5$ cellules dans un volume de 50 $\mu$l de tampon FACS. Dans chaque puits est ajouté soit un anticorps anti-CD1a humain marqué à la fluorescéine (Becton Dickinson) soit un anticorps anti-CD83 humain marqué à la fluorescéine (Becton Dickinson). Après 20 minutes d'incubation à 4°C, les cellules sont lavées trois fois avec 200 $\mu$l de tampon FACS puis sont remises en suspension dans 200 $\mu$l de ce même tampon. L'analyse de l'expression de CD1a versus CD83 est évaluée par FACS. Seules les cellules dendritiques immatures caractérisées par une expression de la molécule CD1a (intensité moyenne de fluorescence (IMF) > 100) et l'absence d'expression de la molécule CD83 ont été utilisées.

- Analyse par cytofluorométrie de l'expression de CD83 sur les CD

Les CD immatures ont été collectées, lavées puis remises en culture en milieu complet à la concentration de 105 cellules dans un volume de 200 $\mu$l dans des plaques de culture 96 puits à fond plat (Costar, Cabridge, USA). Les cellules sont incubées avec rP40s. Après quatre jours de culture, l'expression de la molécule CD83 est évaluée par FACS en utilisant un AC anti-CD83 révélé par un anticorps anti-immunoglobuline de souris marqué à la fluorescéine (Silenus, Hauworth, Australie). Les anticorps isotypiques contrôles utilisés proviennent de Becton Dickinson. Les cellules sont lavées dans du tampon FACS puis réparties dans des puits d'une plaque de culture 96 puits à fond conique à raison de 2 x $10^5$ cellules dans un volume de 50 $\mu$l de tampon FACS. Dans chaque puits est ajouté un anticorps. Après 20 minutes d'incubation à 4°C, les cellules sont lavées trois fois avec 200 $\mu$l de tampon FACS puis sont remises en suspension dans 200 $\mu$l de ce même tampon. L'analyse de l'expression des marqueurs de surface est évaluée par FACS.

Les résultats présentés dans la figure 2 montrent que rP40s induit, de façon dépendante de la dose, l'expression de la molécule CD83 sur une fraction des CD immatures. Des résultats identiques ont été obtenus avec les cellules provenant de deux sujets différents (■ et □).

rP40s induit une production d'IL-12 par les CD humaines

- Evaluation de la production d'interleukine 12 (IL-12) par les CD

Les CD immatures obtenues comme décrit dans l'exemple 4 ont été collectées, lavées puis remises en culture en milieu complet à la concentration de $10^5$ cellules dans un volume de 200 $\mu$l dans des plaques de culture 96 puits à fond plat (Costar, Cabridge, USA). Les cellules sont incubées avec rP40s. Après deux jours de culture, on a déterminé la concentration de l'hétérodimère p40/p35 d'IL-12 active (IL-12 p75) dans les surnageants sans cellules, par un essai d'immunofluorescence par cytométrie de flux (Bioergonomics, St. Paul, MN) selon les recommandations du fabricant (sensibilité de 5 pg/ml).

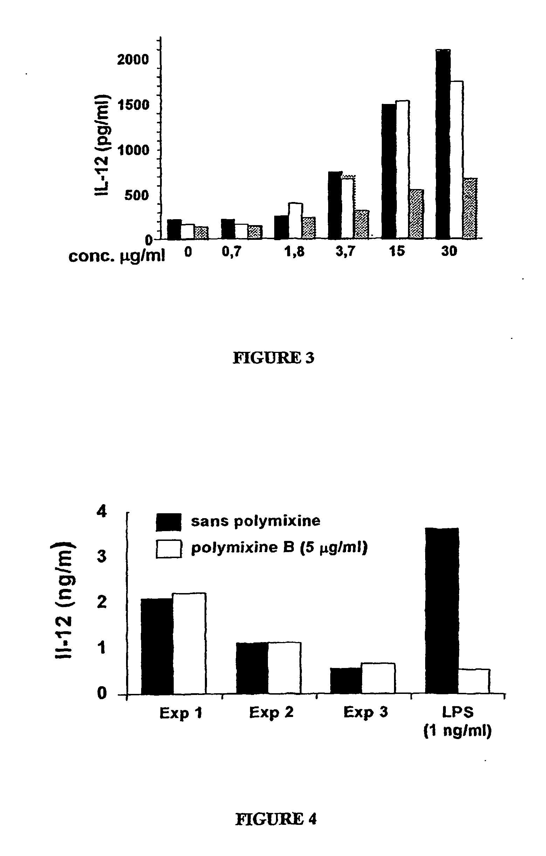

Les résultats présentés dans la figure 3 (exprimés en pg/ml ou en ng/ml) montrent que rP40s induit une production de la dose d'IL-12 biologiquement active par les CD humaines, qui dépend de la concentration en rP40s. Les résultats ont été obtenus avec les cellules provenant de trois sujets différents (■, □ et ▦).

**Exemple 6 : L'effet de rP40s sur la maturation des CD n'est pas dû à des endotoxines contaminantes.**

Les endotoxines et en particulier les lipopolysacharides (LPS) sont des molécules qui induisent une maturation très rapide et importante des CD. Les CD immatures ont donc été exposées à rP40s en présence de polymixine B afin d'exclure que l'effet de rP40s sur la maturation des DC puisse être dû à des endotoxines contaminantes. La polymixine B inhibe l'effet du LPS.

Des CD immatures obtenues à partir de monocytes comme décrit dans l'exemple 5 ont été collectées, lavées puis remises en culture en milieu complet à la concentration de $10^5$ cellules dans un volume de 200 µl dans des plaques de culture 96 puits à fond plat (Costar, Cabridge, USA). Les CD sont incubées avec rP40s ou avec du lipopolysacharide (LPS) (purifié à partir de la souche Escherichia coli isotype 0111 : B4 ; Sigma) en absence ou en présence de 5 µg/ml de sulfate de polymixine B (Sigma). Après deux jours de culture, on mesure le taux d'hétérodimère dTL-12 p40/p35 (IL,-12 p75) biologiquement active dans les surnageants de culture, par ELISA, en utilisant une trousse commerciale (R & D Systems) en suivant les recommandations du fabricant (sensibilité de 0,5 pg/ml).

Les résultats (exprimés en ng/ml) montrent que :

- le LPS induit une production d'IL-12 par les CD,
- l'effet du LPS sur la production d'IL-12 par les CD est inhibé par la polymixine B,
- l'effet de rP40s sur la production d'IL-12 par les CD n'est pas inhibé par la polymixine B (les résultats obtenus en utilisant les cellules issues de 3 sujets différents sont présentés dans la figure 4).

De plus, la polymixine B n'inhibe pas l'expression de la molécule CD83 sur les CD induites par rP40s (données non présentées).

Ainsi, l'ensemble de ces données montre que l'effet de rP40s sur la maturation des CD humaines n'est pas dû à la présence d'endotoxines contaminantes, mais bien que la protéine rP40s, obtenue suivant le procédé selon l'invention possède une activité biologique permettant la maturation des cellules dendritiques.

**Exemple 7 : La molécule rP40s purifiée et renaturée selon l'invention induit une maturation des CD beaucoup plus importante que la molécule rP40.**

L'effet de rP40s et de rP40 a été comparé sur la maturation des CD. La figure 5 montre que rP40s induit une production d'IL-12 beaucoup plus importante que rP40.

Des CD immatures obtenues comme décrit dans l'exemple 5 ont été collectées, lavées puis remises en culture en milieu complet à la concentration de $10^5$ cellules dans un volume de 200 µl dans des plaques de culture 96 puits à fond plat. Les CD sont incubées avec rP40s ou avec rP40. Après deux jours de culture, l'IL-12 bioactive a été dosée dans les surnageants comme décrit dans l'exemple 5.

Les résultats (exprimés en pg/ml) montrent que rP40s est beaucoup plus efficace que rP40 pour induire une production d'IL-12 par les CD.

On peut aussi montrer que rP40S est également beaucoup plus efficace que rP40 pour induire l'expression de CD83 sur les CD immatures (données non présentées).

Ainsi, l'ensemble de ces résultats montre que rP40s. purifiée et rénaturée suivant le procédé décrit dans la présente invention, induit la maturation des CD humaines et possède une activité biologique similaire et supérieure à la protéine rP40 purifiée selon les techniques conventionnelles.

**Exemple 8 : Induction d'anticorps anti TNP par rP40-TNP et rP40s-TNP**

Préparation des conjugués rP40-TNP, rP40s-TNP et KLH-TNP

La solution de TNP-sulfonic acid (Sigma), 25 mM finale, est ajoutée à la solution de protéine (1 mg/ml, dans $Na_2CO_3$ 0,1 M). Trois protéines sont couplées : rP40, rP40s et KLH (Pierce). Après cinq heures d'incubation à température ambiante dans l'obscurité, la solution est dialysée une nuit dans du PBS.

Immunisation des animaux et détermination de la réponse anticorps anti-TNP

100 µg de conjugués sont injectés par voie intrapéritonéale à des souris Balb c (n = 5), à J0 et J21, en présence d'alum. Le sérum est prélevé à J14 et J30. La réponse anticorps anti-haptène (IgG1a et IgG2a anti-TNP) est déterminée par ELISA en utilisant des plaques revêtues avec de la TNP-BSA préparée en utilisant le même protocole que ci-dessus.

La réponse anticorps anti-TNP induite par les conjugués rP40s-TNP est similaire à celle induite par les conjugués rP40-TNP

La figure 6A illustre les réponses anticorps IgG2a anti-TNP induites respectivement par les conjugués anatoxines tétanique-TNP (TT), rP40s-TNP (rP40s) et rP40-TNP (rP40). Les réponses anticorps anti-TNP sont exprimées en densité optique pour une dilution du sérum de 2 500 fois. Comme le montre la figure, les réponses induites par les conjugués rP40s et rP40 sont statistiquement similaires aux deux temps testés. La réponse induite par les conjugués TT est plus précoce.

La figure 6B illustre les réponses anticorps IgG1 anti-TNP induites respectivement par les conjugués anatoxine tétanique-TNP (TT), rP40s-TNP (rP40s) et rP40-TNP (rP40). Les réponses anticorps sont exprimées en µg d'anticorps anti-TNP par ml. La figure montre que, pour ce type d'anticorps, les réponses sont également similaires pour les protéines rP40s et rP40, réponses largement supérieures à celles observées pour les conjugués TT.

La protéine rP40s présente les mêmes propriétés que rP40 pour l'induction d'une réponse anti-haptène.

**Exemple 9 : Induction d'une réponse cytotoxique par des conjugués rP40-ovalbumine et rP40s-ovalbumine**

Préparation des conjugués rP40-ova et rP40s-ova

rP40s et rP40 sont conjugués à l'ovalbumine par couplage au glutaraldéhyde suivant la méthode d'Avrameas (Avrameas et Terninck, Immunochemistry (1969), 6 : 53).

Immunisation des animaux et détermination de la réponse CTL induite

100 µg de conjugués rP40s-ovalbumine (rP40s-ova) et rP40-ovalbumine (rP40-ova) dans 200 µl de PBS sont injectés à des souris C57BL16 femelles (H-2$^b$) âgées de 5 à 8 semaines. Les animaux sont sacrifiés 7 jours après l'immunisation et les rates sont prélevées.
40 x 10$^6$ cellules spléniques sont cultivées en flasques de 25 cm$^2$ dans 10 ml de milieu cDMEM, pendant cinq jours, en présence de 7 x 10$^6$ cellules E.G7 irradiées (4000 rad).

Dans l'essai de cytolyse, les cellules cibles (EL-4, EL-4 pulsées avec le peptide SIINFEKL et E.G7) sont incubées avec 100 µCi/10$^6$ cellules de sodium chromate ($^{51}$Cr) pendant quatre heures à 37°C et lavées trois fois avec du milieu cDMEM avant utilisation. Les cellules effectrices, constituées par les cellules spléniques des cultures de restimulation, sont cultivées avec 10 000 cellules cibles marquées au $^{51}$Cr pendant cinq heures. Après incubation, 100 µl du surnageant des cultures réalisées en triplicate sont prélevées et comptées. Le tableau 1 ci-après représente les résultats obtenus, résultats exprimés en pourcentage de lyse spécifique pour trois rapports cellules effectrices sur cellules cibles : 100:1, 33:1, 11:1.

TABLEAU 1 : Pourcentage de lyse spécifique obtenu dans l'essai de cytolyse

|  | Cellules ELA-peptide | Cellules EL4 seules |
|---|---|---|
| rP40s-ova | 40/20/14 | 5/1/0 |
| rP40-ova | 67/39/20 | 6/6/6 |
| rP40 | 5/5/5 | 5/5/5 |
| ova | 35/27/16 | 36/25/12 |

Les résultats obtenus avec la rP40s sont similaires à ceux obtenus avec la rP40, la capacité des conjugués rP40s à induire une réponse cytotoxique est du même ordre que celle des conjugués rP40.

**Exemple 10 : Test comparatif de P40 soluble (P40S) versus P40 Zwittergent (P40Z) pour l'induction d'une réponse CTL dirigée contre le peptide ELA**

Pour cette expérience, des souris HLA A2/Kb de 6 semaines sont injectées, en sous cutané, à la base de la queue avec un mélange P40Z+ELA ou P40S+ELA à un ratio de 300:50, en absence de tout adjuvant. Le choix du ratio porteur/peptide a été effectué en fonction de données disponibles pour la molécule P40Z et n'est peut être pas le ratio optimal à utiliser pour le mélange P40S.

Dix jours après l'injection, les souris sont sacrifiées et une suspension cellulaire est préparée à partir des ganglions inguinaux et para aortiques qui drainent le site d'injection du produit. Les cellules sont mises en culture et stimulées *in vitro* par addition de cellules EL4 A2/Kb, chargées avec le peptide ELA et irradiées. 7 jours plus tard, une nouvelle stimulation des cellules effectrices, *in vitro,* comme décrit ci-dessus, est effectuée, puis, après 5 nouveaux jours de culture, un essai de cytotoxicité par la méthode du $^{51}$Cr est effectué. Dans cet essai, les cellules cibles EL4 A2/Kb, chargées avec le peptide et marquées au $^{51}$Cr sont mises en présence d'une gamme de cellules effectrices durant 4 heures et la quantité de $^{51}$Cr relarguée est estimée par comptage du rayonnement γ. Des cellules EL4 A2/Kb non chargées avec le peptide et traitées de la même façon sont utilisées comme contrôle de lyse non spécifique.

Pour ces deux types de cellules, le pourcentage de cytotoxicité est calculé à l'aide de la formule :

## 100 X (Lyse de l'essai – Lyse spontanée / Lyse totale – Lyse spontanée).

Deux courbes sont tracées, l'une à partir des données obtenues avec les cellules EL4 A2/Kb non chargées correspond à la lyse non spécifique, l'autre à partir des cellules EL4 A2/Lb chargées avec le peptide. C'est la soustraction de ces deux courbes (représentées dans les figures 7A et 7B) qui donne l'activité du produit.

Les résultats présentés dans les figures 7A et 7B montrent que P40S co-administrée à ELA (figure7B) est capable d'induire la génération d'une réponse cytotoxique anti-peptide de façon comparable à P40Z (figure 7A). Ce résultats est d'autant plus surprenant que, contrairement à P40Z pour lequel de nombreuses mises au point du ratio P40/peptide ont été effectuées pour obtenir ce type de résultat, aucune mise au point n'a été effectuée avec P40S. Il est donc probablement possible d'améliorer notablement ce résultat

## Revendications

1. Procédé de préparation d'une solution purifiée de polypeptide soluble en solvant aqueux en absence de détergent à partir d'une solution purifiée d'un polypeptide hydrophobe contenant un détergent, ledit polypeptide étant une protéine de la membrane externe de bactérie, **caractérisé en ce qu'**il comprend les étapes suivantes :

   a) élimination dudit détergent de ladite solution purifiée contenant un détergent ;
   b) solubilisation du polypeptide obtenu à l'étape a) dans une solution contenant un agent dénaturant choisi parmi l'urée ou le chlorhydrate de guanidine ;
   c) élution en milieu aqueux du polypeptide solubilisé à l'étape b) par tamisage moléculaire sur colonne de chromatographie.

2. Procédé selon la revendication 1, **caractérisé en ce que** ledit polypeptide purifié contenu dans ladite solution purifiée d'un polypeptide contenant un détergent est un polypeptide recombinant.

3. Procédé selon l'une des revendications 1 et 2, **caractérisé en ce que** l'étape a) d'élimination du détergent de ladite solution purifiée est effectuée par précipitation dudit polypeptide en présence d'un alcool organique, de préférence l'éthanol.

4. Procédé selon l'une des revendications 1 à 3, **caractérisé en ce que** les bactéries sont des bactéries Gram-négatives.

5. Procédé selon la revendication 4, **caractérisé en ce que** le polypeptide est la protéine OmpA de *Klebsiella pneumoniae* de séquence SEQ ID No. 2 ou une protéine hydrophobe dont la séquence présente un pourcentage d'identité d'au moins 80 % après alignement optimal avec la séquence SEQ ID No. 2.

**6.** Procédé selon l'une des revendications 1 à 5, pour la modification d'une structure tertiaire de type feuillet β en structure tertiaire de type hélice a.

**7.** Polypeptide de la membrane externe de bactérie, recombinant, renaturé et soluble dans l'eau, obtenu par le procédé selon l'une quelconque des revendications 1 à 6, **caractérisé en ce qu'**il présente une structure tertiaire de type hélice α.

**8.** Polypeptide selon la revendication 7, **caractérisé en ce que** le polypeptide est la protéine OmpA de *Klebsiella pneumoniae* de séquence SEQ ID No. 2 ou une protéine dont la séquence présente un pourcentage d'identité d'au moins 80 % après alignement optimal avec la séquence SEQ ID No. 2.

**9.** Utilisation d'un polypeptide selon la revendication 8, seul ou en tant qu'adjuvant, pour la préparation d'une composition thérapeutique soluble dans l'eau en absence de détergent.

**10.** Utilisation selon la revendication 9, en combinaison avec un antigène ou un haptène, pour la préparation d'un médicament destiné à moduler la réponse immunitaire de l'hôte vis-à-vis dudit antigène ou haptène.

**11.** Utilisation selon la revendication 10, **caractérisée en ce que** l'antigène ou l'haptène est couplé audit polypeptide par liaison covalente.

**12.** Utilisation d'un polypeptide selon l'une des revendications 7 et 8 pour la préparation d'un vaccin, en particulier antiviral, antibactérien ou anticancéreux.

**13.** Composition thérapeutique **caractérisée en ce qu'**elle comprend dans un milieu pharmaceutiquement acceptable au moins un polypeptide selon la revendication 8.

**14.** Composition thérapeutique selon la revendication 13, **caractérisée en ce qu'**elle est exempte de détergent.

**15.** Composition thérapeutique selon la revendication 13 ou 14, **caractérisée en ce que** ledit polypeptide est en combinaison avec un antigène ou un haptène.

**Claims**

**1.** Method for preparing a purified solution of polypeptide soluble in aqueous solvent in the absence of detergent, from a purified solution of a hydrophobic polypeptide containing a detergent, said polypeptide being an outer membrane protein of a bacterium, **characterized in that** it comprises the following steps:

a) removing said detergent from said purified solution containing a detergent;
b) solubilizing the polypeptide obtained in step a) in a solution containing a denaturing agent chosen from urea or guanidine hydrochloride;
c) eluting in aqueous medium the polypeptide solubilized in step b), by molecular sieving on a chromatography column.

**2.** Method according to Claim 1, **characterized in that** said purified polypeptide contained in said purified solution of a polypeptide containing a detergent is a recombinant polypeptide.

**3.** Method according to either of Claims 1 and 2, **characterized in that** step a) for removing the detergent from said purified solution is carried out by precipitation of said polypeptide in the presence of an organic alcohol, preferably ethanol.

**4.** Method according to one of Claims 1 to 3, **characterized in that** the bacteria are Gram-negative bacteria.

**5.** Method according to Claim 4, **characterized in that** the polypeptide is the *Klebsiella pneumoniae* OmpA protein of sequence SEQ ID No. 2 or a hydrophobic protein the sequence of which has a percentage identity of at least 80%, after optimal alignment, with the sequence SEQ ID No. 2.

**6.** Method according to one of Claims 1 to 5, for modifying a tertiary structure of the β-sheet type to a tertiary structure of the α-helix type.

7. Recombinant, renatured and water-soluble polypeptide of the outer membrane of bacterium, obtained using the method according to any one of Claims 1 to 6, **characterized in that** it has a tertiary structure of the α-helix type.

8. Polypeptide according to Claim 7, **characterized in that** the polypeptide is the *Klebsiella pneumoniae* OmpA protein of sequence SEQ ID No. 2 or a protein the sequence of which has a percentage identity of at least 80%, after optimal alignment, with the sequence SEQ ID No. 2.

9. Use of a polypeptide according to Claim 8, alone or as an adjuvant, for preparing a therapeutic composition soluble in water in the absence of detergent.

10. Use according to Claim 9, in combination with an antigen or a hapten, for preparing a medicinal product intended to modulate the host's immune response against said antigen or hapten.

11. Use according to Claim 10, **characterized in that** the antigen or the hapten is coupled to said polypeptide by covalent bonding.

12. Use of a polypeptide according to either of Claims 7 and 8, for preparing a vaccine, in particular an antiviral, antibacterial or anticancer vaccine.

13. Therapeutic composition, **characterized in that** it comprises, in a pharmaceutically acceptable medium, at least one polypeptide according to Claim 8.

14. Therapeutic composition according to Claim 13, **characterized in that** it is free of detergent.

15. Therapeutic composition according to Claim 13 or 14, **characterized in that** said polypeptide is in combination with an antigen or a hapten.

## Patentansprüche

1. Verfahren zur Herstellung einer gereinigten Lösung von in wässrigem Lösemittel in Abwesenheit von Detergens löslichem Polypeptid ausgehend von einer gereinigten Lösung eines hydrophoben Polypeptids, welche ein Detergens enthält, wobei das Polypeptid ein Protein der äußeren Membran eines Bakteriums ist, **dadurch gekennzeichnet, dass** es die folgenden Schritte umfasst:

a) Entfernung des Detergens aus der gereinigten Lösung, welche ein Detergens enthält;
b) Solubilisierung des in Schritt a) erhaltenen Polypeptids in einer Lösung, welche ein Denaturierungsmittel, ausgewählt aus Harnstoff oder Guanidinhydrochlorid, enthält;
c) Elution des in Schritt b) solubilisierten Polypeptids in wässriges Medium durch Molekularsieb-Auftrennung an einer Chromatographiesäule.

2. Verfahren nach Anspruch 1, **dadurch gekennzeichnet, dass** das gereinigte Polypeptid, das in der gereinigten Lösung eines Polypeptids, welche ein Detergens enthält, enthalten ist, ein rekombinantes Polypeptid ist.

3. Verfahren nach einem der Ansprüche 1 und 2, **dadurch gekennzeichnet, dass** der Schritt a) der Entfernung des Detergens aus der gereinigten Lösung durch Ausfällung des Polypeptids in Gegenwart eines organischen Alkohols, vorzugsweise Ethanol, ausgeführt wird.

4. Verfahren nach einem der Ansprüche 1 bis 3, **dadurch gekennzeichnet, dass** die Bakterien gramnegative Bakterien sind.

5. Verfahren nach Anspruch 4, **dadurch gekennzeichnet, dass** das Polypeptid das Protein OmpA von *Klebsiella pneumoniae* mit der Sequenz SEQ ID Nr. 2 oder ein hydrophobes Protein ist, dessen Sequenz einen Prozentsatz von Identität von wenigstens 80% nach optimalem Alignment mit der Sequenz SEQ ID Nr. 2 aufweist.

6. Verfahren nach einem der Ansprüche 1 bis 5 für die Modifizierung einer Tertiärstruktur vom Typ β-Faltblatt zu einer

Tertiärstruktur vom Typ $\alpha$-Helix.

7. Rekombinantes, renaturiertes und in Wasser lösliches Polypeptid der äußeren Membran eines Bakteriums, welches durch das Verfahren nach einem der Ansprüche 1 bis 6 erhalten wird, **dadurch gekennzeichnet, dass** es eine Tertiärstruktur vom Typ $\alpha$-Helix aufweist.

8. Polypeptid nach Anspruch 7, **dadurch gekennzeichnet, dass** das Polypeptid das Protein OmpA von *Klebsiella pneumoniae* mit der Sequenz SEQ ID Nr. 2 oder ein Protein ist, dessen Sequenz einen Prozentsatz von Identität von wenigstens 80% nach optimalem Alignment mit der Sequenz SEQ ID Nr. 2 aufweist.

9. Verwendung eines Polypeptids nach Anspruch 8 allein oder als Hilfsstoff bzw. Adjuvans für die Herstellung einer in Abwesenheit von Detergens in Wasser löslichen therapeutischen Zusammensetzung.

10. Verwendung nach Anspruch 9 in Kombination mit einem Antigen oder einem Hapten für die Herstellung eines Arzneimittels, welches dazu bestimmt ist, die Immunantwort des Wirts gegenüber dem Antigen oder Hapten zu modulieren.

11. Verwendung nach Anspruch 10, **dadurch gekennzeichnet, dass** das Antigen oder das Hapten mit dem Polypeptid durch kovalente Bindung verknüpft ist.

12. Verwendung eines Polypeptids nach einem der Ansprüche 7 und 8 für die Herstellung eines Impfstoffs, insbesondere eines gegen Viren, gegen Bakterien oder gegen Krebs gerichteten Impfstoffs.

13. Therapeutische Zusammensetzung, **dadurch gekennzeichnet, dass** sie in einem pharmazeutisch annehmbaren Medium wenigstens ein Polypeptid nach Anspruch 8 umfasst.

14. Therapeutische Zusammensetzung nach Anspruch 13, **dadurch gekennzeichnet, dass** sie frei von Detergens ist.

15. Therapeutische Zusammensetzung nach Anspruch 13 oder 14, **dadurch gekennzeichnet, dass** das Polypeptid in Kombination mit einem Antigen oder einem Hapten vorliegt.

**FIGURE 1**

**FIGURE 2**

**FIGURE 3**

**FIGURE 4**

**FIGURE 5**

## Dosage IgG2a avec alum

**FIGURE 6A**

**Dosage IgG1 anti TNP avec alum**

FIGURE 6B

FIGURE 7A

FIGURE 7B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5846751 A
- US 5814455 A
- EP 0334278 A
- WO 9527787 A
- WO 9614415 A
- FR 9814007
- FR 9901917
- FR 0000070

**Littérature non-brevet citée dans la description**

- **KYTE ; DOOLITTLE.** *J. Mol. Biol.,* 1982, vol. 157, 105-132
- **SMITH ; WATERMAN.** *Ad. App. Math,* 1981, vol. 2, 482
- **NEDDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **GAP ; BESTFIT ; FASTA ; TFASTA.** *Wisconsin Genetics Software Package*
- **STEINMAN RM. et al.** *Immuno. Rev.,* 1997, vol. 156, 25
- **SELLA M. et al.** *Curr. Opin. Immunol.,* 1997, vol. 9, 10
- **NGUYEN ; COL.** *Gene,* 1998
- **ROSENBUSCH.** heat modifiable protein. *J. Biol. Chem.,* 1974, vol. 249, 8019-8029
- **NAKAMURA ; MIZUSHIMA.** *J. Biochem,* 1976, vol. 80, 1411-1422

LISTE DE SEQUENCES

<110> PIERRE FABRE MEDICAMENT

<120> PROCEDE DE PREPARATION D'UN POLYPEPTIDE SOLUBLE EN SOLVANT AQUEUX EN ABSENCE DE DETERGENT

<130> D18390

<140>
<141>

<150> FR 00 00070
<151> 2000-01-04

<160> 2

<170> PatentIn Ver. 2.1

<210> 1
<211> 1035
<212> ADN
<213> Klebsiella pneumoniae

<220>
<221> CDS
<222> (1)..(1032)

<220>
<223> P40

<400> 1

```
ATG AAA GCA ATT TTC GTA CTG AAT GCG GCT CCG AAA GAT AAC ACC TGG      48
Met Lys Ala Ile Phe Val Leu Asn Ala Ala Pro Lys Asp Asn Thr Trp
 1               5                   10                  15

TAT GCA GGT GGT AAA CTG GGT TGG TCC CAG TAT CAC GAC ACC GGT TTC      96
Tyr Ala Gly Gly Lys Leu Gly Trp Ser Gln Tyr His Asp Thr Gly Phe
                20                  25                  30

TAC GGT AAC GGT TTC CAG AAC AAC AAC GGT CCG ACC CGT AAC GAT CAG     144
Tyr Gly Asn Gly Phe Gln Asn Asn Asn Gly Pro Thr Arg Asn Asp Gln
                 35                  40                  45

CTT GGT GCT GGT GCG TTC GGT GGT TAC CAG GTT AAC CCG TAC CTC GGT     192
Leu Gly Ala Gly Ala Phe Gly Gly Tyr Gln Val Asn Pro Tyr Leu Gly
                50                  55                  60

TTC GAA ATG GGT TAT GAC TGG CTG GGC CGT ATG GCA TAT AAA GGC AGC     240
Phe Glu Met Gly Tyr Asp Trp Leu Gly Arg Met Ala Tyr Lys Gly Ser
65                  70                  75                  80

GTT GAC AAC GGT GCT TTC AAA GCT CAG GGC GTT CAG CTG ACC GCT AAA     288
Val Asp Asn Gly Ala Phe Lys Ala Gln Gly Val Gln Leu Thr Ala Lys
                85                  90                  95
```

13

```
CTG GGT TAC CCG ATC ACT GAC GAT CTG GAC ATC TAC ACC CGT CTG GGC        336
Leu Gly Tyr Pro Ile Thr Asp Asp Leu Asp Ile Tyr Thr Arg Leu Gly
            100             105             110

GGC ATG GTT TGG CGC GCT GAC TCC AAA GGC AAC TAC GCT TCT ACC GGC        384
Gly Met Val Trp Arg Ala Asp Ser Lys Gly Asn Tyr Ala Ser Thr Gly
            115             120             125

GTT TCC CGT AGC GAA CAC GAC ACT GGC GTT TCC CCA GTA TTT GCT GGC        432
Val Ser Arg Ser Glu His Asp Thr Gly Val Ser Pro Val Phe Ala Gly
            130             135             140

GGC GTA GAG TGG GCT GTT ACT CGT GAC ATC GCT ACC CGT CTG GAA TAC        480
Gly Val Glu Trp Ala Val Thr Arg Asp Ile Ala Thr Arg Leu Glu Tyr
145             150             155             160

CAG TGG GTT AAC AAC ATC GGC GAC GCG GGC ACT GTG GGT ACC CGT CCT        528
Gln Trp Val Asn Asn Ile Gly Asp Ala Gly Thr Val Gly Thr Arg Pro
            165             170             175

GAT AAC GGC ATG CTG AGC CTG GGC GTT TCC TAC CGC TTC GGT CAG GAA        576
Asp Asn Gly Met Leu Ser Leu Gly Val Ser Tyr Arg Phe Gly Gln Glu
            180             185             190

GAT GCT GCA CCG GTT GTT GCT CCG GCT CCG GCT CCG GCT CCG GAA GTG        624
Asp Ala Ala Pro Val Val Ala Pro Ala Pro Ala Pro Ala Pro Glu Val
            195             200             205

GCT ACC AAG CAC TTC ACC CTG AAG TCT GAC GTT CTG TTC AAC TTC AAC        672
Ala Thr Lys His Phe Thr Leu Lys Ser Asp Val Leu Phe Asn Phe Asn
            210             215             220

AAA GCT ACC CTG AAA CCG GAA GGT CAG CAG GCT CTG GAT CAG CTG TAC        720
Lys Ala Thr Leu Lys Pro Glu Gly Gln Gln Ala Leu Asp Gln Leu Tyr
225             230             235             240

ACT CAG CTG AGC AAC ATG GAT CCG AAA GAC GGT TCC GCT GTT GTT CTG        768
Thr Gln Leu Ser Asn Met Asp Pro Lys Asp Gly Ser Ala Val Val Leu
            245             250             255

GGC TAC ACC GAC CGC ATC GGT TCC GAA GCT TAC AAC CAG CAG CTG TCT        816
Gly Tyr Thr Asp Arg Ile Gly Ser Glu Ala Tyr Asn Gln Gln Leu Ser
            260             265             270

GAG AAA CGT GCT CAG TCC GTT GTT GAC TAC CTG GTT GCT AAA GGC ATC        864
Glu Lys Arg Ala Gln Ser Val Val Asp Tyr Leu Val Ala Lys Gly Ile
            275             280             285

CCG GCT GGC AAA ATC TCC GCT CGC GGC ATG GGT GAA TCC AAC CCG GTT        912
Pro Ala Gly Lys Ile Ser Ala Arg Gly Met Gly Glu Ser Asn Pro Val
            290             295             300
```

```
ACT GGC AAC ACC TGT GAC AAC GTG AAA GCT CGC GCT GCC CTG ATC GAT     960
Thr Gly Asn Thr Cys Asp Asn Val Lys Ala Arg Ala Ala Leu Ile Asp
305             310             315             320

TGC CTG GCT CCG GAT CGT CGT GTA GAG ATC GAA GTT AAA GGC TAC AAA    1008
Cys Leu Ala Pro Asp Arg Arg Val Glu Ile Glu Val Lys Gly Tyr Lys
                325             330             335

GAA GTT GTA ACT CAG CCG GCG GGT TAA                                1035
Glu Val Val Thr Gln Pro Ala Gly  *
                340
```

<210> 2
<211> 344
<212> PRT
<213> Klebsiella pneumoniae

<220>
<223> P40

<400> 2

```
Met Lys Ala Ile Phe Val Leu Asn Ala Ala Pro Lys Asp Asn Thr Trp
1               5                   10                  15
Tyr Ala Gly Gly Lys Leu Gly Trp Ser Gln Tyr His Asp Thr Gly Phe
            20                  25                  30
Tyr Gly Asn Gly Phe Gln Asn Asn Asn Gly Pro Thr Arg Asn Asp Gln
        35                  40                  45
Leu Gly Ala Gly Ala Phe Gly Gly Tyr Gln Val Asn Pro Tyr Leu Gly
    50                  55                  60
Phe Glu Met Gly Tyr Asp Trp Leu Gly Arg Met Ala Tyr Lys Gly Ser
65                  70                  75                  80
Val Asp Asn Gly Ala Phe Lys Ala Gln Gly Val Gln Leu Thr Ala Lys
            85                  90                  95
Leu Gly Tyr Pro Ile Thr Asp Asp Leu Asp Ile Tyr Thr Arg Leu Gly
            100                 105                 110
Gly Met Val Trp Arg Ala Asp Ser Lys Gly Asn Tyr Ala Ser Thr Gly
        115                 120                 125
Val Ser Arg Ser Glu His Asp Thr Gly Val Ser Pro Val Phe Ala Gly
    130                 135                 140
Gly Val Glu Trp Ala Val Thr Arg Asp Ile Ala Thr Arg Leu Glu Tyr
145                 150                 155                 160
Gln Trp Val Asn Asn Ile Gly Asp Ala Gly Thr Val Gly Thr Arg Pro
            165                 170                 175
Asp Asn Gly Met Leu Ser Leu Gly Val Ser Tyr Arg Phe Gly Gln Glu
            180                 185                 190
```

```
Asp Ala Ala Pro Val Val Ala Pro Ala Pro Ala Pro Ala Pro Glu Val
        195             200         205
Ala Thr Lys His Phe Thr Leu Lys Ser Asp Val Leu Phe Asn Phe Asn
    210             215             220
Lys Ala Thr Leu Lys Pro Glu Gly Gln Gln Ala Leu Asp Gln Leu Tyr
225             230             235             240
Thr Gln Leu Ser Asn Met Asp Pro Lys Asp Gly Ser Ala Val Val Leu
            245             250             255
Gly Tyr Thr Asp Arg Ile Gly Ser Glu Ala Tyr Asn Gln Gln Leu Ser
        260             265             270
Glu Lys Arg Ala Gln Ser Val Val Asp Tyr Leu Val Ala Lys Gly Ile
    275             280             285
Pro Ala Gly Lys Ile Ser Ala Arg Gly Met Gly Glu Ser Asn Pro Val
    290             295             300
Thr Gly Asn Thr Cys Asp Asn Val Lys Ala Arg Ala Ala Leu Ile Asp
305             310             315             320
Cys Leu Ala Pro Asp Arg Arg Val Glu Ile Glu Val Lys Gly Tyr Lys
            325             330             335
Glu Val Val Thr Gln Pro Ala Gly
            340
```